# EUROPÄISCHE PATENTSCHRIFT

(11) **EP 0 952 433 B1**
(45) Veröffentlichungstag und Bekanntmachung des Hinweises auf die Patenterteilung: **20.02.2008**
(21) Anmeldenummer: 99107326.3
(22) Anmeldetag: 20.04.1999
(51) Int. Cl.: G01F 23/292, G01F 25/00

(54) **Vorrichtung zur Überwachung des Füllstands eines Blutreservoirs**
Fluid level sensing system for a blood reservoir
Dispositif de détection de niveau pour un réservoir de sang

(30) Priorität: 22.04.1998 DE 19817995
(43) Veröffentlichungstag der Anmeldung: 27.10.1999
(73) Patentinhaber: Sorin Group Deutschland GmbH, 80939 München (DE)
(72) Erfinder: Hahn, Andreas, Dr.-Ing., 82335 Berg (DE); Schreyer, Johann, 80999 München (DE)
(74) Vertreter: HOFFMANN EITLE

(56) Entgegenhaltungen:
- EP-A- 0 619 476
- DE-A- 4 314 249
- GB-A- 1 030 205
- US-A- 3 851 181
- US-A- 5 414 778
- US-A- 5 665 061

## Beschreibung

Die Erfindung betrifft eine Vorrichtung zur Überwachung des Füllstands eines Blutreservoirs.

Aus EP 0 361 023 A1 ist ein Füllstandsmeßgerät für Blutreservoire bekannt, mit dem ein bestimmter Füllstand des Vorratsbehälters überwacht werden kann. In Höhe des zu überwachenden Füllstands ist außen an der Wand des Behälters als Sensor ein kapazitives Bauteil angebracht, dessen elektrische Eigenschaften sich füllstandsabhängig ändern und das elektrisch in die Schaltung eines Oszillators so integriert ist, daß in Abhängigkeit vom Über- oder Unterschreiten des Füllstandes der Oszillator schwingt bzw. nicht schwingt. Beim Durchschreiten des vorgegebenen Füllstands wird die für das Schwingen des Oszillators maßgebliche Phasen- oder Amplitudenbedingung verändert. Die Änderung des Schwingzustands des Oszillators wird ausgewertet und beim Unterschreiten des kritischen Füllstands ein Anzeigesignal ausgelöst.

Um die Überwachung des Füllstands von dem Material des Vorratsbehälters weitgehend unabhängig zu machen und um eine höhere Auflösung zu erreichen, wird in DE 195 16 789 A1 eine Vorrichtung zur Überwachung des Füllstands eines Vorratsbehälters vorgeschlagen, bei der ein Mikrowellensensor in geringem Abstand zur Innenwand des Behälters angeordnet ist und durch die Behälterwand in das Innere des Behälters abstrahlt. Solange für den Behälter kein elektrisch leitendes Material verwendet wird, ist der Einfluss der Behälterwand gering. Insbesondere wenn die Dielektrizitätskonstante des Behältermaterials sehr viel kleiner als die des Füllmediums ist, ist der Einfluss auf die Mikrowellen vernachlässigbar und lässt keine Probleme entstehen. Das Behältermaterial kann verändert werden, ohne dass eine Anpassung des Sensors vorgenommen werden müsste.

Bei beiden Überwachungsvorrichtungen ist jedoch der Sensor so ausgelegt, dass nur das kritische Niveau genau erfasst wird, was im Ausgangssignal des Sensors zu einem vergleichsweise scharfen Übergang führt. Insbesondere bei der Einbindung des Sensors in einen Regelkreis kann dies zu unerwünschten Effekten führen, die mit entsprechendem Aufwand im Regler ausgeglichen werden müssen.

Die sehr genaue Definition des Detektionsniveaus führt ferner dazu, dass die Sensoren ebenso genau montiert werden müssen, was oft Probleme bereitet. Ein einmal montierter Sensor der bekannten Art wird in der Regel so fixiert, dass er nicht mehr abgelöst werden kann, um ein Verrutschen oder Abfallen sicher auszuschliessen. Dies bedeutet aber, dass ein einmal angebrachter Sensor nicht wieder abgelöst werden kann, um erneut angebracht zu werden, wenn der Sensor versehentlich oberhalb oder unterhalb des zu überwachenden Füllstands befestigt wurde. Dadurch wird die Handhabung erschwert, was insbesondere bei einer Verwendung im medizinischen Bereich unerwünscht ist.

Überdies führt der scharfe Übergang im Bereich des Überwachungsniveaus dazu, dass an der Behälterwand anhaftendes Füllmedium zu Fehlern führen kann, die aufgrund der Konzeption der Sensoren kaum automatisch erfasst und ausgeglichen werden können.

Daneben ist aus US 3.851.818 ein Blutreservoir bekannt, bei dem drei Sensoren drei unterschiedliche Pegel überwachen, um bei Erreichen, Überschreiten oder Unterschreiten der Pegel Signale auszulösen, die die Benutzer auf den Füllstand des Blutreservoirs hinweisen. Die einzelnen Sensoren umfassen ein lichtempfindliches Element und Dioden zur Beleuchtung des Messniveaus.

Aus US 5.665.061 ist eine Vorrichtung zur Beimischung eines Koagulationshemmers in ein Blutreservoir bekannt, bei der zahlreiche Sensoren die gesamte Füllhöhe des Blutreservoirs in einzelne Volumina unterteilen, so dass auf der Basis des in dem Blutreservoir ermittelten Blutvolumens eine entsprechende Menge des Koagulationshemmers zugefügt werden kann.

Ferner ist aus dem Bereich der Abfüllanlagen bekannt, Videokameras für die Erfassung des Füllstands von Behältern, in der Regel Flaschen vorzusehen, wobei das von der Videokamera aufgezeichnete Bild einer Bildbearbeitung unterworfen wird, um den Füllstand der Flaschen zu ermitteln. Vorrichtungen dieser Art sind beispielsweise in US 5.414,778 und DE 43 14 249 beschrieben.

Vor diesem Hintergrund liegt der Erfindung die Aufgabe zugrunde, eine Vorrichtung zur Überwachung des Füllstands eines Blutreservoirs anzugeben, mit der die Einhaltung eines vorgegebenen Füllstandes zuverlässig überwacht werden kann, die einfach gehandhabt werden kann und bei der das Überwachungsniveau einfach verändert werden kann.

Gelöst wird diese Aufgabe durch eine Vorrichtung zur Überwachung des Füllstandes eines Blutreservoirs mit den Merkmalen des Patentanspruchs 1. Vorteilhafte Ausgestaltungen ergeben sich aus den Unteransprüchen.

Durch den Einsatz eines Bildsensors wird erreicht, daß die Überwachung über einen größeren Bereich erfolgt, wodurch mehrere Vorteile erzielt werden.

Zum einen wird ein scharfer Übergang des Sensorsignals vermieden, da als Sensorsignal das Ausgangsbildsignal des Bildsensors zur Verfügung steht, das Informationen über das gesamte von dem Bildsensor erfaßte Bild enthält und nicht auf einen schmalen Bereich in der unmittelbaren Nähe des zu überwachenden Füllstandsniveaus reduziert. Dabei ist zu beachten, daß für den erfindungsgemäßen Bildsensor entscheidend und ausreichend ist, daß er sich senkrecht zur Grenzfläche zwischen Blut und Luft ausreichend erstreckt. In einer Richtung parallel zur Grenzfläche kann die Erstreckung minimal sein, beispielsweise nur eine Pixelreihe eines CCD-Sensors ausmachen.

Zum anderen kann das gesamte Ausgangssignal des Bildsensors verwendet werden, um Fehler zu erfassen, beispielsweise durch an der Behälterwand anhaftendes Füllmedium.

Ferner ist die Montage des Sensors einfacher, da der gesamte Bereich des Bildsensors für die Erfassung des Füllstandes zur Verfügung steht. Geringfügige Verschiebungen bei der Montage können bei der Auswertung des Bildsignals ausgeglichen werden.

Dadurch bietet sich überdies die grundsätzliche Möglichkeit, den zu überwachenden Füllstandspegel im nachhinein zu verschieben, ohne daß der Sensor verschoben werden muß, da bei der Auswertung eine entsprechende Änderung vorgenommen werden kann.

Im Hinblick auf die Überwachung des Füllstandes eines Blutreservoirs ist von besonderer Bedeutung, daß ein Bildsensor keinerlei schädliche Auswirkungen auf das Blut haben kann.

Im folgenden wird die Erfindung anhand eines bevorzugten Ausführungsbeispiels genauer beschrieben, das in den Zeichnungen dargestellt ist.
- Fig. 1: zeigt eine Gesamtansicht einer erfindungsgemäßen Überwachungsvorrichtung;
- Fig. 2: zeigt in vergrößerter und geschnittener Darstellung den Bildsensor des Ausführungsbeispiels gemäß Fig. 1; und
- Fig. 3: zeigt das von dem Bildsensor erfaßte Bild der Außenwand des Blutreservoirs.

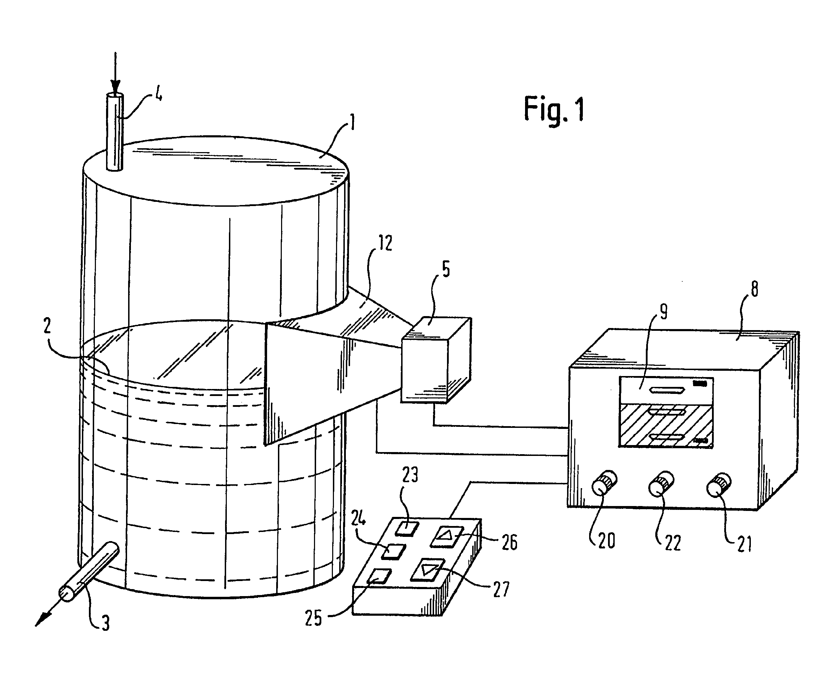

Fig. 1 zeigt schematisch ein Blutreservoir 1, in dem eine gewünschte Blutmenge 2 bevorratet ist, die über einen Abfluß 3 entnommen werden kann, um direkt oder indirekt einem Patienten wieder zugeführt zu werden, und die über einen Zufluß 4 ergänzt wird, indem Blut von dem Patienten oder einem anderen Blutvorrat direkt oder indirekt in das Blutreservoir 1 zugeführt wird. Dabei wird angestrebt, die Blutmenge 2 bei einem vorgegebenen Füllstand zu halten. In jedem Fall muß aber ein Unterschreiten eines minimalen Füllstands verhindert werden, da andernfalls die Gefahr besteht, daß das dem Patienten zugeführte Blut Luftblasen enthält. Ebenso muß ein Überschreiten eines maximalen Füllstands verhindert, damit die bevorratete Blutmenge 2 nicht zu groß wird oder das Blutreservoir 1 überläuft.

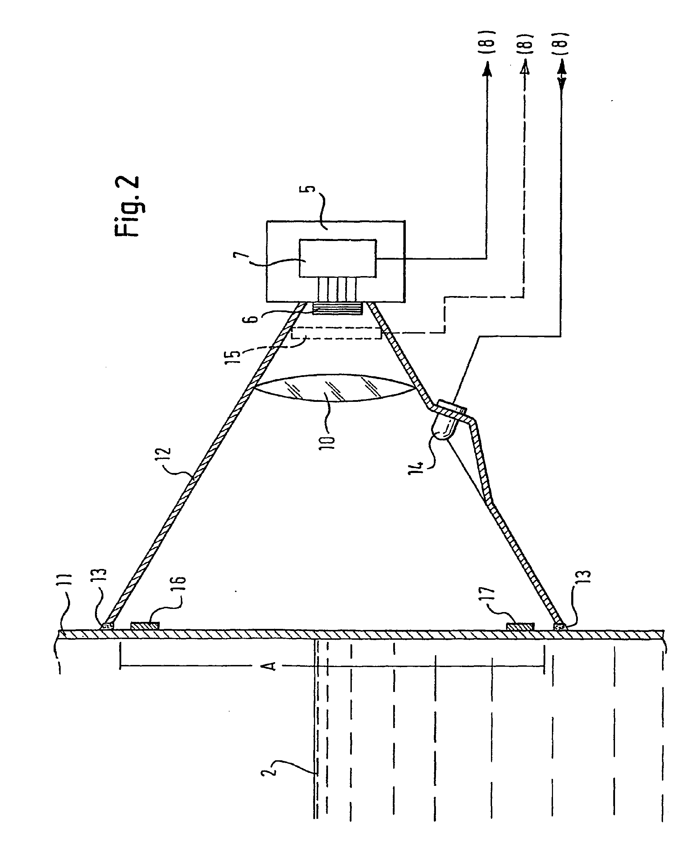

Dazu besitzt das in Fig. 1 gezeigte Ausführungsbeispiel der erfindungsgemäßen Überwachungsvorrichtung einen Bildsensor 5, der in Höhe des zu überwachenden Füllstandes an dem Blutreservoir 1 angebracht ist. Wie sich aus Fig. 2 ergibt, besitzt der Bildsensor 5 einen optischen Erfassungsbereich 6, beispielsweise ein CCD-Feld, und eine elektronische Schaltung 7, die überlicherweise in den Bildsensor integriert ist. Ein auf der Oberfläche des optischen Erfassungsbereichs 6 erzeugtes Bild wird von der in den Bildsensor integrierten Schaltung 7 in ein Ausgangsbildsignal des Bildsensors 5 umgewandelt, das den Bildinhalt wiedergibt. Das Ausgangsbildsignal steht als Sensorausgangssignal zur Verfügung und wird einer Auswerteeinheit 8 zugeführt, die den Bildinhalt auf der Basis des Sensorsignals zur Bestimmung des Füllstands des Blutreservoirs 1 auswertet. Vorteilhaft ist die Auswerteeinheit 8, wie in Fig. 1 gezeigt, mit einer Anzeigeeinrichtung 9 ausgestattet, die das von dem Bildsensor erfaßte Bild für einen Benutzer der erfindungsgemäßen Vorrichtung anzeigt.

Wie in Fig. 2 gezeigt, ist eine optische Abbildungseinrichtung 10, beispielsweise eine Linse, vor dem optischen Erfassungsbereich 6 des Bildsensors 5 derart angeordnet, daß ein hinreichend großer Abschnitt A der Außenwand 11 des Blutreservoirs 1 auf dem optischen Erfassungsbereich 6 des Bildsensors 5 abgebildet wird. Die Größe des abzubildenden Abschnitts A der Außenwand 11 ist abhängig von der konkreten Gestaltung der Überwachungsvorrichtung und den gestellten Anforderungen. Jedoch kann in der Regel davon ausgegangen werden, daß ein Abschnitt mit einer Erstreckung von 10 bis 200 mm senkrecht zur Füllstandsgrenzfläche der bevorrateten Blutmenge 2 abgebildet werden sollte.

Der Bildsensor 5 ist bei dem in Fig. 1 und 2 gezeigten Ausführungsbeispiel am Blutreservoir 1 mittels eines pyramidenstumpfförmigen Trichters 12 angebracht, an dessen zulaufendem Ende der Bildsensor 5 angeordnet ist und der sich zur Außenwand 11 des Blutreservoirs 1 hin öffnet. An den Kanten des Trichters 12, die der Außenwand 11 des Blutreservoirs zugewandt sind, sind Klebeelemente 13 vorgesehen, die den Trichter 12 an der Außenwand 11 des Blutreservoirs 1 fixieren. Diese Kanten des Trichter 12 sind vorteilhafterweise an die Form der Außenwand 11 des Blutreservoirs 1 angepaßt, wie in Fig. 1 erkennbar ist. Der Trichter 12 kann zusätzlich oder alternativ zu den Klebeelementen 13 mit Hilfe eines Gurtes, der um das Blutreservoir gelegt wird, an dem Blutreservoir fixiert werden. In einer weiteren Gestaltung kann der Trichter 12 zusammen mit dem daran befestigten Bildsensor 5 an die Außenwand 11 des Blutreservoirs 1 mit Hilfe eines Stativs o.ä. angedrückt werden.

In einer Seitenwand des Trichters 12 ist eine Lichtquelle 14, beispielsweise eine Leuchtdiode angeordnet, die auf den erfaßten Abschnitt der Außenwand 11 des Blutreservoirs 1 ausgerichtet ist. Das von der Lichtquelle emittierte Licht trifft auf die Außenwand und führt zu einer Veränderung des von der Außenwand erfaßten Bildes, beispielsweise durch Überstrahlung o.ä. In Fig. 2 durch gestrichelte Darstellung nur angedeutet ist ein Verschluß 15, der vor dem optischen Erfassungsbereich 6 des Bildsensors 5 angeordnet ist und der im geschlossenen Zustand die Erzeugung eines Bildes der Außenwand 11 auf dem Bildsensor 5 verhindert. Die Lichtquelle bzw. der Verschluß sind mit der Auswerteeinheit verbunden und werden durch diese angesteuert.

Fig. 2 zeigt ferner zwei Markierungen 16 und 17, die an der Außenwand 11 des Blutreservoirs 1 angebracht sind. Dabei handelt es sich beispielsweise um die in Fig. 2 dargestellten Klebestreifen. Die Markierungen können jedoch auch in der Außenwand 11 des Blutreservoirs 1 angeordnet sein; diese Gestaltung ist jedoch weniger flexibel, da der Benutzer die Lage der Markierungen nicht bestimmen kann. Zumindest die dem Bildsensor 5 zugewandte Oberfläche der Markierungen 16 und 17 besitzt ein Reflexionsvermögen, das sich deutlich von dem Reflexionsvermögen der Außenwand 11 des Blutreservoirs 1 unterscheidet, und zwar unabhängig davon, ob sich aus der Sicht des Bildsensors 5 hinter der Markierung Blut befindet oder nicht. Aufgrund des von der Umgebung verschiedenen Reflexionsvermögens, aber auch aufgrund ihrer Gestalt eignen sich die Markierungen zur Erkennung mittels Bildverarbeitung.

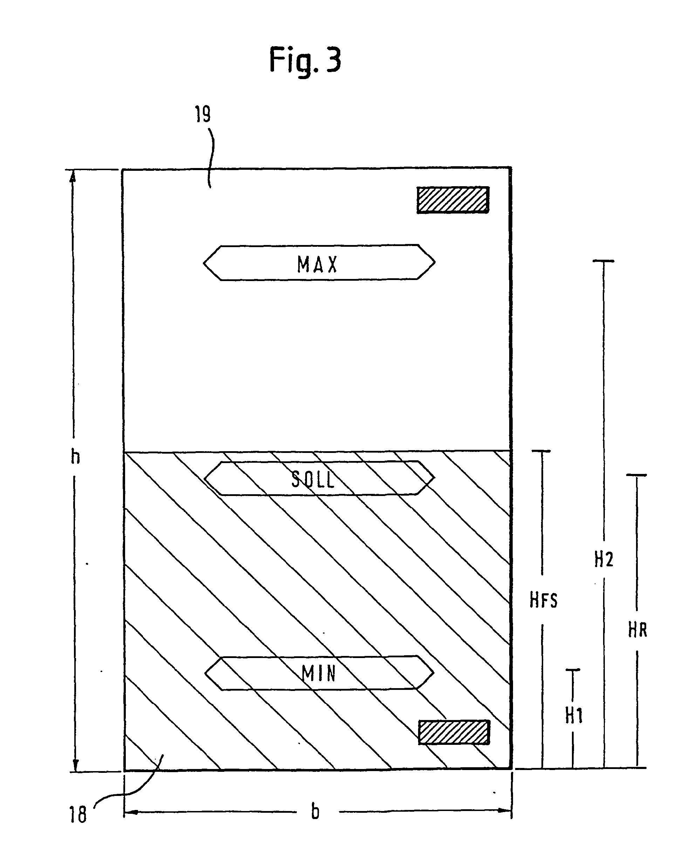

In Fig. 3 ist ein Beispiel eines auf dem Bildsensor erzeugten Bildes der Außenwand 11 des Blutreservoirs 1 schematisch dargestellt. Das Bild hat die Breite b und die Höhe h, die beide bei CCD-Bildsensoren üblicherweise in Pixel angegeben werden. Wie sich aus dieser Darstellung ergibt, ist in dem Bild ein erster Bereich 18, in dem das Blutreservoir mit Blut gefüllt ist und der in Fig.3 den unteren Teil ausmacht, deutlich von einem zweiten Bereich 19 unterscheidbar, in dem das Blutreservoir nicht mit Blut gefüllt ist und der in Fig.3 den oberen Teil umfaßt. Die Auswerteeinheit 8 kann damit anhand der Bildinformation, die durch das Sensorausgangssignal an die Auswerteeinheit geliefert wird, den Füllstand durch Erfassung der Stelle des Übergangs vom ersten Bereich 18 zum zweiten Bereich 19 und die dazu in dem Bild korrespondierende Höhe HFS bestimmen. Dieser Wert kann dann mit den Vorgabewerten verglichen werden.

Der minimale Füllstand wird durch einen Minimalwert festgelegt, der in dem Bild einer Höhe H1 entspricht, wie in Fig. 3 gezeigt, und der von einem Benutzer mit Hilfe einer ersten Eingabeeinrichtung 20 für den minimalen Füllstand in die Auswerteeinheit 8 eingegeben werden kann. Dementsprechend wird der maximale Füllstand durch einen Maximalwert festgelegt, der in dem Bild einer Höhe H2 entspricht, wie in Fig. 3 gezeigt, und der von dem Benutzer mit Hilfe einer zweiten Eingabeeinrichtung 21 für den maximalen Füllstand in die Auswerteeinheit 8 eingegeben werden kann. Zwischen diesen beiden Werten liegt ein Regelwert, der in dem Bild einer Höhe HR entspricht, wie in Fig. 3 gezeigt, und der von dem Benutzer mit Hilfe einer dritten Eingabeeinrichtung 22 für den Regelfüllstand in die Auswerteeinheit 8 eingegeben werden kann. Der Regelwert entspricht dem Füllstand, bei dem die Befüllung des Blutreservoirs vorzugsweise gehalten werden soll, um den aber Schwankungen zulässig sind. Der minimale und der maximale Füllstand sind demgegenüber kritisch, so daß ein Unter- bzw. Überschreiten sofort zu einer Alarmierung des Benutzers und/oder zur Auslösung einer automatischen Gegenmaßnahme führt. Anstelle der Eingabe des Regelwertes durch den Benutzer kann auch vorgesehen sein, daß die Auswerteeinheit 8 anhand des Minimalwertes und Maximalwertes, d.h. anhand des minimalen und des maximalen Füllstands einen Regelwert zwischen den beiden vom Benutzer eingegebenen Werten festlegt. Die Eingabe der beiden Werte kann auch so erfolgen, daß durch die Auswerteeinheit je ein Vorgabewert für den Minimalwert und für den Maximalwert vorgegeben wird, den der Benutzer in einem festgelegten Bereich variieren kann. In jedem Fall wird erreicht, daß der Benutzer den Bildbereich, der zur Überwachung des Füllstands untersucht wird, verändert werden kann, so daß beispielsweise bei einer ungenauen Anbringung des Bildsensors an dem Blutreservoir eine Anpassung vorgenommen werden kann. Schließlich können alle drei Werte fest vorgegeben sein; in diesem Fall muß bei der Anbringung des Bildsensors 5 an dem Blutreservoir 1 größere Sorgfalt aufgewendet werden und vorzugsweise eine Fixierung eingesetzt werden, die ein Verschieben des Bildsensors 5 erlaubt.

Bei den Eingabeeinrichtungen für den Minimalwert, den Maximalwert und den Regelwert kann es sich um die in Fig. 1 gezeigten Drehknöpfe 20, 21 und 22 handeln. Jedoch können auch andere Bedienelemente vorgesehen sein, beispielsweise die in Fig. 1 gezeigten Tasten 23 bis 25. Diese Tasten umfassen Auswahltasten 23 bis 25, mit denen einer der Werte für die Eingabe ausgewählt wird. Beispielsweise kann durch Drücken der Taste 23 der Maximalwert, durch Drücken der Taste 24 der Regelwert und durch Drücken der Taste 25 der Minimalwert für die Eingabe ausgewählt werden. Anschließend wird die Größe des zuvor für die Eingabe ausgewählten Wertes mit Hilfe der Pfeiltasten 26 und 27, vorzugsweise ausgehend von einem Vorgabewert, festgelegt. Schließlich kann auch ein Computer vorgesehen sein, der als Eingabeeinrichtung für diese Werte dient und dessen Tastatur und/oder dessen Zeigeeinrichtung für die Eingabe der Werte vorgesehen sind. In diesem Fall kann vorteilhafterweise der mit dem Computer verwendete Monitor als Anzeigeeinrichtung für das vom Bildsensor erfaßte Bild dienen. Vorteilhaft führt die Veränderung der Werte durch den Benutzer sofort zu einer Veränderung in der Anzeigeeinrichtung 9 bzw. dem Computermonitor, so daß der Benutzter eine unmittelbare Bestätigung und Kontrollmöglichkeit erhält.

Wie in Fig. 2 gezeigt, ist bei dem Ausführungsbeispiel eine Leuchtdiode 14 oder ähnliche Lichtquelle als Bildveränderungsgenerator vorgesehen. Die Leuchtdiode 14 ist auf den abgebildeten Abschnitt der Außenwand 11 des Blutreservoirs 1 ausgerichtet. Die Leuchtdiode 14 wird von der Auswerteeinheit 8 intermittierend angesteuert, so daß das auf dem Bildsensor erzeugte Bild entsprechend intermittierend verändert, beispielsweise überbelichtet oder überstrahlt wird. Es handelt sich um eine provozierte Veränderung des Bildes, die durch die Leuchtdiode 14 generiert wird, die aber auch bei sonst unbekanntem Bildinhalt erkennbar bzw. von der Auswerteeinheit feststellbar ist. Auf diese Weise ist es möglich, die Funktion des Bildsensors 5 mit Hilfe der Auswerteeinheit 8 zu prüfen, da synchron zur Ansteuerung der Leuchtdiode 14 eine Veränderung des Bildes erfaßt werden muß. Kann die Auswerteeinheit 8 trotz Ansteuerung der Leuchtdiode 14 keine Veränderung des Bildes erfassen, löst die Auswerteeinheit 8 einen Alarm aus, der den Benutzer auf den Defekt hinweist. Damit wird bei der erfindungsgemäßen Überwachungsvorrichtung mit Hilfe des Bildveränderungsgenerators die gewünschte Erstfehlersicherheit erzielt.

Wie in Fig. 2 gezeigt, kann als Bildveränderungsgenerator auch ein elektrisch angesteuerter Verschluß 15 dienen, der vor dem Bildsensor angeordnet ist und der im geschlossenen Zustand die Abbildung der Außenwand 11 auf dem optischen Erfassungsbereich 6 des Bildsensor 5 zumindest teilweise verhindert. Als elektrisch angesteuerter Verschluß 15 kommt eine Flüssigkristallzelle oder Matrix in Frage, aber auch ein mechanischer Verschluß, der elektrisch von der Auswerteeinheit ansteuerbar ist.

Um die Lage des Bildsensors zu überprüfen, sind bei dem Ausführungsbeispiel gem. Fig. 2 Markierungen 16 und 17 vorgesehen, die an oder in der Außenwand des Blutreservoirs angebracht sind. Dabei handelt es sich um Markierungen, die von der Auswerteeinheit in dem vom Bildsensor erfaßten Bild gem. Fig. 3 aufgrund ihrer Gestalt und ihres Reflexionsvermögens erkannt werden können. Die Markierungen können auf die Außenwand in Form von Klebestreifen aufgeklebt werden, sie können aber auch in die Wand des Blutreservoirs integriert sein.

Unmittelbar nach der Anbringung des Bildsensors 5 an dem Blutreservoir 1 und nach dem Einschalten der Auswerteeinheit 8, überprüft die Auswerteeinheit 8 das Bild auf das Vorhandensein von zumindest einer Markierung 16 und 17. Wird keine Markierung festgestellt, informiert die Auswerteeinheit den Benutzer, der darauf hingewiesen wird, daß ohne zumindest eine Markierung im erfaßten Bild die Überwachungsvorrichtung nicht betrieben werden kann. Wird eine Markierung festgestellt, speichert die Auswerteeinheit die Lage der Markierung in dem Bild ab, um später wiederholt das Vorhandensein der Markierung zu überprüfen, deren Lage erneut zu festzustellen und mit der ursprünglichen Lage zu vergleichen. Weicht die zu einem späteren Zeitpunkt festgestellte Lage der Markierung von der ursprünglichen Lage ab oder ist keine Markierung mehr feststellbar, wird der Benutzer informiert, da dies ein Hinweis auf einen Fehler ist. Der Bildsensor 5 kann an der Außenwand 11 des Blutreservoirs 1 verrutscht oder vom Blutreservoir 1 abgefallen sein oder die Markierung kann sich verschoben oder abgelöst haben. Auch ein Defekt des Bildsensors 5 führt zu einer Fehlermeldung an den Benutzer.

Wenn zwei Markierungen vorgesehen werden, können diese auch als obere und untere Grenze für den Maximalwert bzw. den Minimalwert verwendet werden, so daß die Auswerteeinheit 8 nur eine Verschiebung des Maximalwertes und des Minimalwertes bis zu diesen Grenzen zuläßt. Die beiden Markierungen können auch den Maximalwert und den Minimalwert unmittelbar festlegen, wodurch die Markierungen quasi die Funktion der zuvor erwähnten Eingabeeinrichtungen für diese Werte übernehmen. Insbesondere wenn die Markierungen als Klebestreifen realisiert werden, bleibt dem Benutzer die Möglichkeit, diese beiden Werte festzulegen. In ähnlicher Weise kann mit einer dritten Markierung der zuvor erwähnte Regelwert vom Benutzer festgelegt werden.

Auf der Basis des Überwachungsergebnisses kann die Auswerteeinheit 8 den Benutzer alarmieren und/oder regelnd in den extrakorporalen Blutkreislauf eingreifen.

## Patentansprüche

1. Vorrichtung zur Überwachung des Füllstands eines Blutreservoirs (1), mit
- einem Sensor, der an der Außenwand (11) des Blutreservoirs anbringbar ist, und
- einer Auswerteeinheit (8), die mit dem Sensor verbunden ist und der von dem Sensor ein Sensorausgangssignal zuführbar ist, und welche für die Auswertung des zugeführten Sensorsignals zur Überwachung des Füllstandes geeignet ist;
**dadurch gekennzeichnet, dass**
- der Sensor (5) ein Bildsensor ist, der einen optischen Erfassungsbereich (6) aufweist, und dass die Vorrichtung
- eine optische Abbildungseinrichtung (10), die einen Abschnitt (A) der Außenwand (11) des Blutreservoirs auf dem optischen Erfassungsbereich des Bildsensors (5) abbildet, und
- einen austeuerbaren Bildveränderungsgenerator (14, 15) umfasst, der eine derartige Veränderung in dem auf dem optischen Erfassungsbereich (6) des Bildsensors (5) erzeugten Bild erzeugt, dass mit der provozierten Veränderung des Bildes die Funktion des Bildsensors mit Hilfe der Auswerteeinheit geprüft werden kann, da synchron zur Ansteuerung des Bildveränderungsgenerators eine Veränderung des Bildes erfasst werden muss.

2. Vorrichtung nach Anspruch 1, **dadurch gekennzeichnet, dass** der Bildveränderungsgenerator eine Lichtquelle (14) ist, die auf den Abschnitt (A) der Außenwand (11) des Blutreservoirs (11), der auf den optischen Erfassungsbereich (6) des Bildsensors (5) abgebildet wird, ausgerichtet ist.

3. Vorrichtung nach Anspruch 2, **dadurch gekennzeichnet, dass** die Lichtquelle zumindest eine Leuchtdiode (14) umfasst.

4. Vorrichtung nach einem der Ansprüche 1 bis 3, **dadurch gekennzeichnet, daß** der Bildveränderungsgenerator ein Verschluss (15) ist, der vor dem optischen Erfassungsbereich (6) des Bildsensors (5) angeordnet ist.

5. Vorrichtung nach Anspruch 4, **dadurch gekennzeichnet, dass** der Verschluss ein mechanischer Verschluss oder ein Flüssigkristallelement ist.

6. Vorrichtung nach einem der vorangegangenen Ansprüche,
**dadurch gekennzeichnet, dass** der Bildsensor (5) an einem Anbringungselement (12) angeordnet ist, das an der Außenwand (11) des Blutreservoirs (1) anbringbar ist.

7. Vorrichtung nach Anspruch 6,
**dadurch gekennzeichnet, dass** das Anbringungselement ein pyramidenstupfförmiger Trichter (12) ist, an dessen zulaufendem Ende der Bildsensor (5) angeordnet ist.

8. Vorrichtung nach Anspruch 7,
**dadurch gekennzeichnet, dass** an den Kanten des sich aufweitenden Endes des pyramidenstumpfförmigen Trichters (12) Klebeelemente (13) vorgesehen sind.

9. Vorrichtung nach Anspruch 7 oder 8,
**dadurch gekennzeichnet, dass** die Kanten des sich aufweitenden Endes des pyramidenstumpfförmigen Trichters (12) an die Form der Außenwand (11) des Blutreservoirs (1) angepasst sind.

10. Vorrichtung nach einem der vorangegangenen Ansprüche
**dadurch gekennzeichnet, dass** die Auswerteeinheit (8) eine Anzeigeeinrichtung (9) zur Anzeige des von dem Bildsensor (5) erfassten Bildes umfasst.

11. Vorrichtung nach einem der vorangegangenen Ansprüche,
**dadurch gekennzeichnet, dass** die Auswerteeinheit (8) Eingabeeinrichtungen (20, 21, 22; 23 bis 27) für die Eingabe von Vorgabewerten, insbesondere eines Minimalwerts, eines Maximalwerts und/oder eines Regelwerts umfasst.

12. Vorrichtung nach Anspruch 11,
**dadurch gekennzeichnet, dass** die Eingabeeinrichtungen Drehknöpfe (20, 21, 22) sind.

13. Vorrichtung nach Anspruch 11,
**dadurch gekennzeichnet, dass** die Eingabeeinrichtungen Tasten (23 bis 27) sind, von denen eine erste Gruppe (23 bis 25) für die Auswahl des einzugebenden Wertes und eine zweite Gruppe (26, 27) für die Festlegung des Wertes vorgesehen sind.

14. Vorrichtung nach einem der vorangegangenen Ansprüche
**dadurch gekennzeichnet, dass** die Auswerteeinheit (8) auf der Basis des Sensorausgangssignals den Füllstand des Blutreservoirs bestimmt und mit den Vorgabewerten vergleicht.

15. Vorrichtung nach einem der vorangegangenen Ansprüche,
**dadurch gekennzeichnet, dass** die Auswerteeinheit (8) auf der Basis des Sensorausgangssignals das Vorhandensein von zumindest einer Markierung (16, 17) überprüft.

16. Vorrichtung nach Anspruch 15,
**dadurch gekennzeichnet, dass** die zu überprüfende Markierung sich aufgrund ihrer Gestalt und/oder ihres Reflektionsvermögens vom übrigen Bildinhalt abhebt.

17. Vorrichtung nach Anspruch 15 oder 16,
**dadurch gekennzeichnet, dass** die Markierung (16, 17) auf die Außenwand (11) des Blutreservoirs (1) aufgeklebt ist.

## Claims

1. Device for monitoring the filling level of a blood reservoir (1) having
- a sensor which is attachable to the outside wall of the blood reservoir and
- an evaluating unit (8) which is connected to the sensor and to which from the sensor a sensor output signal is suppliable and which is suitable for the evaluation of the supplied sensor signal for monitoring the filling level,
**characterised in that**
- the sensor (5) is an image sensor having an optical detection region (6) and that the device
- comprises an optical imaging device (10) which projects a section (A) of the outside wall (1) of the blood reservoir on the optical detection region of the image sensor (5) and
- an actuable image alteration generator (14, 15) which generates such an alteration in the image generated on the optical detection region (6) of the image sensor (5), and that with the induced alteration of the image the operation of the image sensor can be tested with the aid of the evaluating unit since synchronously with the actuation of the image alteration generator an alteration of the image must be detected.

2. Device according to claim 1, **characterised in that** the image alteration generator is a light source (14) which is directed at the section (A) of the outside wall (11) of the blood reservoir (1) that is projected onto the optical detection region (6) of the image sensor (5).

3. Device according to claim 2, **characterised in that** the light source comprises at least one light-emitting diode (14).

4. Device according to any of claims 1 to 3, **characterised in that** the image alteration generator is a shutter (15) that is arranged in front of the optical detection region (6) of the image sensor (5).

5. Device according to claim 4, **characterised in that** the shutter is a mechanical shutter or a liquid crystal element.

6. Device according to any of the preceding claims, **characterised in that** the image sensor (5) is arranged on an attaching element (12) that is attachable to the outside wall (11) of the blood reservoir (1).

7. Device according to claim 6, **characterised in that** the attaching element is a truncated pyramidal funnel (12) on whose tapered end the image sensor (5) is arranged.

8. Device according to claim 7, **characterised in that** on the edges of the widening end of the truncated pyramidal funnel (12) adhesive bonding elements (13) are provided.

9. Device according to claim 7 or 8, **characterised in that** the edges of the widening end of the truncated pyramidal funnel (12) are adapted to the shape of the outside wall (11) of the blood reservoir (1).

10. Device according to any of the preceding claims, **characterised in that** the evaluating unit (8) comprises a display device (9) for displaying the image captured by the image sensor (5).

11. Device according to any of the preceding claims, **characterised in that** the evaluating unit (8) comprises input devices (20, 21, 22; 23 to 27) for the input of values specified in advance, in particular a minimum value, a maximum value and/or a control value.

12. Device according to claim 11, **characterised in that** the input devices are rotary knobs (20, 21, 22).

13. Device according to claim 11, **characterised in that** the input devices are keys (23 to 27) of which a first group (23 to 25) is provided for the selection of the value to be input and a second group (26, 27) for fixing the value.

14. Device according to any of the preceding claims, **characterised in that** the evaluating unit (8) determines the filling level of the blood reservoir on the basis of the sensor output signal and compares it with the specified values.

15. Device according to any of the preceding claims, **characterised in that** the evaluating unit (8) checks the presence of at least one mark (16, 17) on the basis of the sensor output signal.

16. Device according to claim 15, **characterised in that** the mark to be checked stands out from the remaining image content on account of its shape and/or its reflectivity.

17. Device according to claim 15 or 16, **characterised in that** the mark (16, 17) is stuck to the outside wall (11) of the blood reservoir (1).

## Revendications

1. Dispositif de surveillance du niveau de remplissage d'un réservoir de sang (1), comprenant :
- un capteur, susceptible d'être monté sur la paroi extérieure (11) du réservoir de sang, et
- une unité d'évaluation (8), reliée au capteur et à laquelle un signal de sortie de capteur, provenant du capteur, est susceptible d'être amené, et qui convient pour l'évaluation du signal de capteur amené, aux fins de surveillance du niveau de remplissage,
**caractérisé en ce que**
- le capteur (5) est un capteur d'image présentant une zone de détection (6) optique, et **en ce que** le dispositif comprend
- un dispositif d'imagerie optique (10), imageant un tronçon (A) de la paroi extérieure (11) du réservoir de sang sur la zone de détection optique du capteur d'image (5), et
- un générateur de modification d'image (14, 15) susceptible d'être commandé, produisant, dans la zone de détection (6) optique du capteur d'image (5), une modification de l'image produite, telle que, avec la modification provoquée de l'image, on puisse vérifier le fonctionnement du capteur d'image à l'aide de l'unité d'évaluation, du fait qu'une modification de l'image doit être détectée de façon synchrone à la commande du générateur de modification d'image.

2. Dispositif selon la revendication 1, **caractérisé en ce que** le générateur de modification d'image est une source lumineuse (14), orientée sur le tronçon (A) de la paroi extérieure (11) du réservoir de sang (1), imagé sur la zone de détection (6) optique du capteur d'image (5).

3. Dispositif selon la revendication 2, **caractérisé en ce que** la source lumineuse comprend au moins une photodiode (14).

4. Dispositif selon l'une des revendications 1 à 3, **caractérisé en ce que** le générateur de modification d'image est un obturateur (15), disposé en amont de la zone de détection (6) optique du capteur d'image (5).

5. Dispositif selon la revendication 4, **caractérisé en ce que** l'obturateur est un obturateur mécanique, ou un élément à cristal liquide.

6. Dispositif selon l'une des revendications précédentes, **caractérisé en ce que** le capteur d'image (5) est disposé sur un élément de montage (12) susceptible d'être monté sur la paroi extérieure (11) du réservoir de sang (1).

7. Dispositif selon la revendication 6, **caractérisé en ce que** l'élément de montage est un entonnoir (12) en forme de tronc de pyramide, à l'extrémité allant en s'effilant duquel est disposé le capteur d'image (5).

8. Dispositif selon la revendication 7, **caractérisé en ce que**, sur les bords de l'extrémité, allant en s'élargissant, de l'entonnoir (12) en forme de tronc de pyramide sont prévus des éléments adhésifs (13).

9. Dispositif selon la revendication 7 ou 8, **caractérisé en ce que** les bords de l'extrémité, allant en s'élargissant, de l'entonnoir (12) en forme de tronc de pyramide sont adaptés à la forme de la paroi extérieure (11) du réservoir de sang (1).

10. Dispositif selon l'une des revendications précédentes, **caractérisé en ce que** l'unité d'évaluation (8) comprend un dispositif d'affichage (9), pour afficher l'image appréhendé par le capteur d'image (5).

11. Dispositif selon l'une des revendications précédentes, **caractérisé en ce que** l'unité d'évaluation (8) comprend des dispositifs d'introduction (20, 21, 22 ; 23 à 27) pour l'introduction de valeurs d'affectation, en particulier d'une valeur minimale, d'une valeur maximale et/ou d'une valeur de régulation.

12. Dispositif selon la revendication 11, **caractérisé en ce que** les dispositifs d'introduction sont des boutons rotatifs (20, 21, 22).

13. Dispositif selon la revendication 11, **caractérisé en ce que** les dispositifs d'introduction sont des touches (23 à 27), dont un premier groupe (23 à 25) est prévu pour la sélection de la valeur à introduire et un deuxième groupe (26, 27) est prévu pour la fixation de la valeur.

14. Dispositif selon l'une des revendications précédentes, **caractérisé en ce que** l'unité d'évaluation (8), sur la base du signal de sortie de capteur, détermine le niveau de remplissage du réservoir de sang et le compare aux valeurs d'affectation.

15. Dispositif selon l'une des revendications précédentes, **caractérisé en ce que** l'unité d'évaluation (8), sur la base du signal de sortie de capteur, vérifie la présence d'au moins un marquage (16, 17).

16. Dispositif selon la revendication 15, **caractérisé en ce que** le marquage à vérifier ressort du reste du contenu de l'image, du fait de sa forme et/ou de son pouvoir réfléchissant.

17. Dispositif selon la revendication 15 ou 16, **caractérisé en ce que** le marquage (16, 17) est collé sur la paroi extérieure (11) du réservoir de sang (1).
